# EUROPÄISCHE PATENTSCHRIFT

(11) **EP 1 371 988 B1**
(45) Veröffentlichungstag und Bekanntmachung des Hinweises auf die Patenterteilung: **27.04.2005**
(21) Anmeldenummer: 03012913.4
(22) Anmeldetag: 06.06.2003
(51) Int. Cl.: G01N 33/74

(54) **Verfahren zur Testung des hormonellen Effekts von Substanzen**
Assay for the determination of the hormonal effect of compounds
Méthode de détermination de l'effet hormonal de molécules

(30) Priorität: 12.06.2002 DE 10226675
(43) Veröffentlichungstag der Anmeldung: 17.12.2003
(73) Patentinhaber: Schering Aktiengesellschaft, 13353 Berlin (DE)
(72) Erfinder: Wolf, Siegmund, Dr., 07745 Jena (DE); Obendorf, Maik, Dr., 99423 Weimar (DE); Meyer, Rene, 08468 Reichenbach (DE)
(74) Vertreter: Störle, Christian, Dr.

(56) Entgegenhaltungen:
- EP-A- 1 255 113
- SCOTT H S ET AL: "IDENTIFICATION AND CHARACTERIZATION OF TWO PUTATIVE HUMAN ARGININE METHYLTRANSFERASES (HRMT1L1 AND HRMT1L2)" GENOMICS, ACADEMIC PRESS, SAN DIEGO, US, Bd. 48, Nr. 3, 15. März 1998 (1998-03-15), Seiten 330-340, XP001030144 ISSN: 0888-7543 -& DATABASE EMBL [Online] EBI; 15. April 1998 (1998-04-15) SCOTT HS ET AL: "H. sapiens mRNA for arginine methyltransferase (HSRNAAM)" retrieved from EMBL Database accession no. X99209 XP002258092
- MCKENNA N ET AL: "Nuclear receptor coregulators: Cellular and molecular biology" ENDOCRINE REVIEWS, BALTIMORE, MD, US, Bd. 20, Nr. 3, Juni 1999 (1999-06), Seiten 321-344, XP002234409
- LEO C ET AL: "The SRC family of nuclear receptor coactivators" GENE, ELSEVIER BIOMEDICAL PRESS. AMSTERDAM, NL, Bd. 245, Nr. 1, März 2000 (2000-03), Seiten 1-11, XP004202955 ISSN: 0378-1119
- QI CHAO ET AL: "Identification of protein arginine methyltransferase 2 as a coactivator for estrogen receptor alpha" JOURNAL OF BIOLOGICAL CHEMISTRY, Bd. 277, Nr. 32, 9. August 2002 (2002-08-09), Seiten 28624-28630, XP002258082 ISSN: 0021-9258

## Beschreibung

Die Erfindung bezieht sich auf ein Verfahren zur Testung des hormonellen Effekts von Substanzen, ein Verfahren zur Bestimmung von Störungen im Co-Modulationsmechanismus zwischen Androgenrezeptor und dem Co-Aktivator Arginin-Methyltransferase (HSRNAAM).

Bei der Beurteilung von Substanzen für eine mögliche pharmazeutische Anwendung ist es allgemein üblich, diese Substanzen auf eine eventuelle hormonelle Wirkung, insbesondere auf eine möglicherweise vorhandene androgene oder antiandrogene Aktivität, zu prüfen. Bei der Verabreichung pharmakologisch wirksamer Substanzen sind Kenntnisse über hormonelle Effekte, insbesondere androgene oder antiandrogene Effekte, dieser Substanzen in manchen Fällen von Bedeutung, da sie beim Patienten unerwünschte Nebenwirkungen hervorrufen können. Zur Prüfung der hormonellen Wirkung von Substanzen kommen insbesondere Verfahren zum Einsatz, bei denen die Fähigkeit der Substanzen gemessen wird, an die Hormonrezeptoren zu binden und deren Transkriptionsaktivität zu aktivieren.

Kenntnisse über hormonelle Effekte von Substanzen sind aber nicht nur bei potentiellen Pharmaka von Interesse, sondern auch bei nicht-pharmazeutischen Substanzen, da von vielen, in der Umwelt vorhandenen Substanzen angenommen wird, daß sie bei Teilen der Bevölkerung eine androgene oder antiandrogene bzw. estrogene oder antiestrogene Aktivität aufweisen können. Möglicherweise wird dadurch eine unerwünschte, schädliche Wirkung hervorgerufen.

Es besteht also ein ganz erhebliches Bedürfnis für ein Verfahren und für ein für die Durchführung des Verfahrens geeignetes Mittel, mit dem in zuverlässiger, empfindlicher, einfacher, kostengünstiger und schneller Weise eine Aussage über den hormonellen Effekt von Substanzen getroffen werden kann. Die bisher bekannten Verfahren genügen dem nicht.

Der vorliegenden Erfindung liegt deshalb die Aufgabe zu Grunde, ein Verfahren und dazu geeignete Mittel bereitzustellen, mit denen in zuverlässiger, empfindlicher, einfacher, kostengünstiger und schneller Weise eine Aussage über den hormonellen Effekt der zu testenden Substanzen getroffen werden kann.

Erfindungsgemäß wird dies in überraschender Weise erreicht durch ein Verfahren zur Testung des hormonellen Effekts, insbesondere des androgenen oder antiandrogenen Effekts, von Substanzen, bei dem
a) Zellen, die mit zwei Vektoren transfektiert sind, wobei der eine Vektor DNA, die für ein nukleäres Rezeptor-Protein oder ein Fragment davon, insbesondere ein humanes nukleäres Rezeptor-Protein oder ein Fragment davon, kodiert, und der andere Vektor DNA enthält, die den HSRNAAM Co-Modulator oder ein Fragment davon kodiert, der Substanz ausgesetzt werden und
b) die Transkriptionsaktivität, die der nukleäre Rezeptor oder dessen Fragment in Anwesenheit von HSRNAAM oder dessen Fragment auslöst, und/oder der Einfluß der Substanz auf die Interaktion zwischen dem Rezeptor oder dessen Fragment und HSRNAAM oder dessen Fragment durch Protein-Protein-Interaktion oder Protein-Protein-DNA-Interaktion gemessen wird.

Es wurde überraschenderweise gefunden, daß mit dem erfindungsgemäßen Verfahren in zuverlässiger, empfindlicher, einfacher, schneller und kostengünstiger Weise getestet werden kann, ob Substanzen, die beispielsweise umweltrelevant oder pharmakologisch von Interesse sein können, einen hormonellen Effekt, insbesondere einen androgenen oder antiandrogenen Effekt, ausüben.

Im erfindungsgemäßen Verfahren werden mit einem Vektor transformierte Zellen eingesetzt, wobei die Vektoren DNA aufweisen, die für ein nukleäres Rezeptor-Protein oder ein Fragment davon kodiert.

Die Superfamilie der nukleären Rezeptoren (NRs), zu der mehr als 50 verschiedene Proteine gehören, ist eine Gruppe verwandter Transkriptionsfaktoren, die die Transkription des jeweiligen Zielgens als Reaktion auf spezifische Liganden, z. B. Hormone, steuern. Die Familie kann nach bestimmten Charakteristika, wie z.B. Dimerisationsstatus, Art des Liganden oder Struktur des DNA-Reaktionselements, in mehrere Subfamilien unterteilt werden (Beato et al., 2000, Human Reproduct. Update, 6, 225-236). Charakteristisches Merkmal der NRs ist die übereinstimmende Struktur der funktionellen Domäne (mit den Bezeichnungen A bis F) mit einer stark variablen, nur schwach konservierten N-terminalen Region mit autonomer konstitutiver Aktivierungsfunktion (AF-1), einer stark konservierten DNA-Bindungsdomäne (DBD), die für die Erkennung von speziellen DNA-Reaktionselementen verantwortlich ist und aus zwei Zinkfinger-Motiven besteht, einer variablen Scharnierdomäne und einer konservierten multifunktionalen C-terminalen Ligandenbindungsdomäne (LBD) mit Dimerisations- und Liganden-abhängiger Transaktivierungsfunktion (AF-2). Im Anschluß daran folgt die am weitesten C-terminal gelegene Region, deren Funktion nicht bekannt ist und die bei Rezeptoren wie z.B. PR (Progesteron-Rezeptor), PPAR (Peroxisomproliferator-aktivierter Rezeptor) und RXR (Retinoid-X-Rezeptor) fehlt (Mangelsdorf & Evans, 1995; Cell, 83, 841-850; Robyr et al., 2000, Mol. Endocrinol., 14, 329-347). Für einige NRs (z.B. den Androgen-Rezeptor (AR)) wurde nachgewiesen, daß die N-terminale Region in der Lage ist, mit der C-terminalen Region zu interagieren (Brinkmann et al., 1999, J. Steroid Biochem. and Mol. Biol., 69, 307-313). Steroidhormonrezeptoren, wie z.B. Estrogen- (ER), Progesteron- (PR), Glukokortikoid- (GR), Mineralokortikoid- (MR) und Androgenrezeptoren (AR), binden steroidale Liganden, die sich von Pregnenolon ableiten, wie die Progestine, die Estrogene, die Glukokortikoide und die Mineralokortikoide, sowie die Androgene. Die Ligandenbindung aktiviert den Rezeptor und steuert die Expression entsprechender Zielgene.

Wie vorstehend ausgeführt wurde, werden in Schritt a) des erfindungsgemäßen Verfahrens Zellen verwendet, die ferner einen Vektor enthalten, der für den Co-Modulator HSRNAAM oder ein Fragment davon kodierende DNA enthält

Die sog. Co-Modulatoren sind eine Klasse von Proteinen, die bei der Aktivierung (Co-Aktivatoren) bzw. Repression (Co-Repressoren) der Gentranskription als Brückenmoleküle zwischen dem Transkriptionsinitiationskomplex und den NRs dienen (McKenna et al., 1999, Endocr. Rev., 20, 321-347). Ein Co-Aktivator muß fähig sein, die Rezeptorfunktion zu verstärken und in Anwesenheit eines Agonisten mit der Aktivierungsdomäne von NRs direkt zu interagieren. Er muß auch mit dem basalen Transkriptionsapparat interagieren, und schließlich darf er nicht selbst die basale Transkriptionsaktivität verstärken. Die meisten Co-Modulatoren interagieren mit Hilfe eines oder mehrerer LXXLL-Motiv(en) (NR-Boxes) mit der AF-2-Domäne von NRs, jedoch wurden auch einige Co-Modulatoren beschrieben, die mit anderen NR-Regionen interagieren (Ding et al., 1998, Mol. Endocrinol., 12, 302-313). Chao Qui et al. beschreibt das Protein PRMT2 (protein arginine methyltransferase 2) als Coactivator für ERα (JBC Papers in Press, 30. Mai 2002, Manuskript M201053200).

Im erfindungsgemäßen Verfahren wird der mit Arginin-Methyltransferese (HSRNAAM) bezeichnete Co-Modulator oder insbesondere das die Aminosäuren 271 bis 433 aufweisende Fragment von HSRNAAM eingesetzt. Die cDNA-Sequenz wurde beschieben (Genbank X99209) und weist 433 Aminosäuren auf (Scott et al., 1998, Genomics, 48, 330-349). DH5 alpha E. coli Klone, die ein für die Aminosäuren 1 - 433 bzw. 271 - 433 von HSRNAAM kodierende Plasmid enthalten, wurden-unter DSM 15041 bzw. DSM 15042 am 5. Juni 2002 bei der Deutschen Sammlung für Mikroorganismen und Zellkulturen hinterlegt.

Bei Verwendung dieser Proteine kann in besonders zuverlässiger, empfindlicher, einfacher, kostengünstiger und schneller Weise das erfindungsgemäße Verfahren durchgeführt werden. Ferner weisen die HSRNAAM-Fragmente, insbesondere das die Aminosäuren 271 bis 433 aufweisende Fragment von HSRNAAM, den Vorteil auf, daß sie leichter handhabbar und klonierbar sind, aber noch die funktiellen Eigenschaften von HSRNAAM aufweisen.

Bei HSRNAAM handelt es sich um einen Co-Aktivator für den humanen Androgenrezeptor und andere nukleäre Rezeptoren, der die Interaktion zwischen einem Androgen und dem Rezeptor verstärkt. Die Sequenz von HSRNAAM ist bereits in der Genbank X99209 beschrieben; allerdings ist dort keine Interaktion mit nukleären Rezeptoren, insbesondere dem AR, angegeben. Die Erfindung beruht auf der überraschenden Erkenntnis, daß nukleäre Rezeptoren, insbesondere AR, einerseits und HSRNAAM andererseits interagieren sowie die AR-vermittelte Transaktivierung verstärkt wird. HSRNAAM ist ein Protein, das als Co-Mediator fungiert, indem es nach Bindung von Steroiden an den nukleären Rezeptor den Transkriptionseffekt verstärkt oder reprimiert und darüber hinaus die Bindung und Aktivierung des nukleären Rezeptors an Moleküle fördert, denen früher keine hormonelle Wirkung zugeschrieben wurde.

Das Protein HSRNAAM stellt einen Co-Aktivator für den Androgenrezeptor und weitere nukleäre Rezeptoren dar, wie Estrogenrezeptor α, Estrogenrezeptor β, Progesteronrezeptor A, Progesteronrezeptor B, Glukokortikoidrezeptor, Mineralokortikoidrezeptor, Schilddrüsenhormonrezeptor, Vitamin-D-Rezeptor, Peroxisomproliferator-aktivierter Rezeptor, Retinsäurerezeptor, Retinoid-X-Rezeptor und Orphan-Rezeptoren; im erfindungsgemäßen Verfahren werden diese Rezeptoren bevorzugt eingesetzt, da damit die vorstehenden Vorteile des erfindungsgemäßen Verfahrens besonders günstig erreicht werden können.

In erfindungsgemäßen. Verfahren können auch Vektoren, die für Fragmente vorstehender Proteine kodieren, eingesetzt werden. Unter dem Ausdruck "Fragmente" im Zusammenhang mit vorstehenden Proteinen werden solche verstanden, die eine Aminosäure oder mehrere Aminosäuren weniger als die Proteine in voller Länge und noch die funktionellen Eigenschaften eines nukleären Rezeptors oder eines Co-Modulators aufweisen.

Wie bereits vorstehend ausgeführt wurde, werden im erfindungsgemäßen Verfahren in Schritt a) Zellen eingesetzt, die mit zwei Vektoren transfektiert sind, die für spezielle Proteine kodierende DNA enthalten. Diese Zellen sind also in der Lage, diese beiden unterschiedlichen Proteine zu exprimieren.

Vorzugsweise sind die Zellen etablierte Zelllinien und/oder eukaryotische Zellen, insbesondere Prostatazellen, Nervenzellen, Gliazellen, Fibroblasten, Blutzellen, Osteoblasten, Osteoklasten, Hepatozyten, Epithelzellen oder Muskelzellen. Mit den etablierten Zelllinien kann das erfindungsgemäße Verfahren besonders kostengünstig und schnell durchgeführt werden. Bei Verwendung eukaryotischer Zellen, insbesondere vorstehend aufgeführter eukaryotischer Zellen, können mit dem erfindungsgemäßen Verfahren vorteilhafterweise besonders aussagekräftige Ergebnisse erhalten werden.

In einer bevorzugten Ausführungsform des erfindungsgemäßen Verfahrens werden eukaryotische Expressionsvektoren eingesetzt, z.B. pCMX, pCMV oder pSG5. Bei Verwendung dieser Vektoren, insbesondere in Verbindung mit vorstehenden etablierten Zelllinien und/oder eukaryotischen Zellen, kann das erfindungsgemäße Verfahren besonders günstig und schnell durchgeführt werden und es werden besonders aussagekräftige Ergebnisse erhalten.

Der Fachmann kennt Verfahren und dazu notwendige Materialien, um die für die vorstehenden Proteine kodierende DNA in einen Vektor zu inserieren, diesen dann in die Zellen einzubringen und die so erhaltenen Zellen unter geeigneten Kulturbedingungen zu kultivieren, damit sie diese Proteine exprimieren können.

Gemäß Schritt b) des erfindungsgemäßen Verfahrens wird die Transkriptionsaktivität gemessen, die der nukleäre Rezeptor oder dessen Fragment in Anwesenheit des Co-Modulators oder dessen Fragment auslöst. Dies kann beispielsweise durch Detektion eines Reportergens erfolgen.

Reportergene sind Gene oder Genfragmente, die mit anderen Genen oder regulatorischen Sequenzen gekoppelt werden, um die Aktivität dieser Sequenzen nachweisbar zu machen. Reportergene erzeugen Genprodukte, die möglichst einfach nachweisbar sind, z. B. photometrisch durch Farbreaktionen. Häufig verwendete Reportergene sind das Gen für β-Galactosidase, das Gen für die alkalische Phosphatase, das Gen für Chloramphenicol-Acetyltransferase, das Gen für die Catechol-Dioxygenase, das Gen für das "green fluorescent protein" sowie verschiedene Luciferase-Gene, die die Zellen zum Leuchten bringen können.

Solche Reportergene können ebenfalls mittels Vektoren, insbesondere eukaryotischer Expressionsvektoren, in die Zellen eingebracht werden. Beispiel eines Vektors, der für ein Reportergen kodierende DNA enthält, ist der Vektor MMTV-Luciferase, der zur Messung der androgenen Wirkung von Substanzen eingesetzt wird.

Substanzen mit einem hormonellen Effekt, insbesondere einem androgenen/antiandrogenen Effekt, sind dann an der erhöhten bzw. verminderten Aktivität des Reportergens erkennbar.

Die Messung des Einflusses der Testsubstanz auf die Interaktion zwischen dem Rezeptor oder dessen Fragment und dem Co-Modulator oder dessen Fragment kann auch durch Bestimmung der Protein-Protein-Interaktion, z. B. durch Doppelt-Hybrid Systeme, Immunpräzipitation, GST-Pull-down-Assays, FRET-Analayse und ABCD-Assays sowie durch Bestimmung der Protein-Protein-DNA-Interaktion, wie durch Gelretardationsassays, erfolgen.

Es wurde ferner gefunden, daß HSRNAAM sehr gut als Indikator androgenbedingter Erkrankungen verwendet werden kann. Relevante androgenbedingte Erkrankungen, wie z.B. Prostatakrebs, erektile Dysfunktion, Infertilität, Glatzenbildung, Akne oder Hypogonadismus, sowie Androgenresistenzsyndrome, wie z.B. die testikuläre Feminisierung, beruhen auf Defekten im Co-Modulationsmechanismus zwischen AR und HSRNAAM. Eine Möglichkeit bei Patienten mit derartigen Störungen besteht somit in der Messung der relativen Konzentrationen von AR und HSRNAAM, wobei diese Messung günstigerweise in Körperflüssigkeiten, Körperzellen oder Körpergewebe extrakorporal erfolgt. Dies ist möglich durch Anwendung quantitativer Verfahren zur Messung der relativen Menge beider Moleküle bei dem jeweiligen Patienten, bei denen beispielsweise Antikörper sowohl gegen AR als auch gegen HSRNAAM oder Nukleinsäuresonden gegen deren mRNA eingesetzt werden können. Es gibt mehrere Verfahren zur Messung dieser komparativen Raten, die dem Fachmann bekannt sind; auch kennt er hierzu geeignete Materialien und Vorrichtungen, wie Radioimmunassay, ELISA-Test, Immunfärbung, RT-PCR, Western-Blot oder Northern-Blot, DNA-Chip oder Protein-Chip. Darüber hinaus ist es möglich, mit Hilfe der HSRNAAM-cDNA in üblicher Weise Sonden für ein PCR-Assay zu konstruieren, mit dem sich bei bestimmten Patienten Mutationen der normalen DNA-Sequenz nachweisen oder Transkripte für den Northem Blot Assay bzw. eine DNA für Insitu-Hybridisierungsassays generieren lassen.

Das gemessene Verhältnis von AR zu HSRNAAM kann dabei größer oder kleiner als das bei Gesunden sein. Der bei Gesunden vorliegende Normalwert kann in einfacher Weise beispielsweise dadurch bestimmt werden, daß das Verhältnis von AR zu HSRNAAM bei einer Vielzahl von gesunden Probanden gemittelt wird. Durch den Vergleich des Normalwertes mit dem ermittelten Verhältnis von AR zu HSRNAAM bei dem zu untersuchenden Patienten kann festgestellt werden, ob der Wert für das ermittelte Verhältnis größer oder kleiner als der Normalwert ist.

Da die Konzentration von HSRNAAM und/oder AR im Gewebe unterschiedlich sein kann, z.B. ist die Konzentration von HSRNAAM in den Vorkammern des Herzens, im Knochenmarkes, Thymus und Uterus sehr groß, wohingegen sie in Leber, Lunge und Prostata geringer ist, sind die unterschiedlichen Gewebekonzentrationen für die Auswertung zu berücksichtigen, d.h. der Testwert und der Normalwert sollen vom gleichen Gewebe stammen.

Eine andere Möglichkeit zur Bestimmung von Defekten im Co-Modulationsmechanismus zwischen AR und HSRNAAM kann darin bestehen, nur die Konzentration von HSRNAAM zu messen, wobei man dabei davon ausgeht, daß die AR-Konzentration zumindestens annähemd konstant ist. Ist hierbei eine geringere als die normale HSRNAAM-Konzentration gemessen worden, heißt dies, daß sich das Verhältnis von AR zu HSRNAAM verschoben hat, was wiederum als Hinweis auf eine Störung im Co-Modulationsmechanismus hinweist.

Es ist also möglich, mit einer HSRNAAM spezifischen Sonde Änderungen in der HSRNAAM-Expression und damit im Verhältnis zu AR zu bestimmen. Solche Änderungen können bei verschiedenen Krankheitsbildern ursächlich involviert sein oder als Folgeerscheinung auftreten.

Derartigen Messungen des AR/HSRNAAM-Verhältnisses liegt die überraschende und auf die Auffindung und Charakterisierung von HSRNAAM als Co-Modulator beruhende Erkenntnis zugrunde, daß beispielsweise ein Androgenresistenzsyndrom auf einer Störung des Gleichgewichts zwischen AR- und HSRNAAM-Prävalenz in den Zielzellen beruhen kann. Zuviel HSRNAAM könnte zu einer Überempfindlichkeit des AR-Systems führen, so daß es auf Moleküle reagiert, die normalerweise keinen androgenen Effekt haben. Umgekehrt führt das Fehlen oder eine Fehlfunktion von HSRNAAM auf allen Ebenen zur Androgenresistenz. Der Nachweis von zu viel HSRNAAM bei einem Patienten spräche für die Anwendung von Mitteln zur Down-Regulation, wie z.B. Antisense- oder ähnlichen Medikamenten, um unter klinischen Bedingungen den HSRNAAM-Titer bei dem jeweiligen Patienten zu reduzieren. Dasselbe kann durch Moleküle erreicht werden, die in der Lage sind, die Interaktion zwischen AR und HSRNAAM zu hemmen. Hat ein Patient zu wenig HSRNAAM, kann man ihm HSRNAAM-cDNA, -Protein oder -DNA über verschiedene an sich bekannte Mechanismen zuführen, um auf diese Weise den Titer des aktiven HSRNAAM zu erhöhen. Möglich ist auch eine Anhebung der Konzentration oder der Aktivität von HSRNAAM durch niedrigmolekulare Arzneimittel oder durch Stimulation der Eigensynthese mit Hilfe spezifischer HSRNAAM-Promoter-Proteine.

Die Erfindung wird unter Bezugnahme auf die folgenden Abbildungen näher erläutert, wobei
Abbildung 1 eine schematische Darstellung des Androgenrezeptors mit Kennzeichnung der von den Aminosäuren 325 bis 919 reichenden Androgenrezeptor-Domäne (AR 2) ist, die zur Interaktion mit HSRNAAM in Anwesenheit von Androgen fähig ist;
Abbildung 2 die Gewebsverteilung von HSRNAAM in menschlichen Geweben darstellt;
Abbildung 3 die Wirkung von HSRNAAM (AS 1-433) als Co-Modulator mit dem Androgenrezeptor in PC3-ARwt-Zellen zeigt, und
Abbildung 4 die Interaktion des HSRNAAM-Fragmentes (AS 271-433) oder SRC-1a mit dem Androgenrezeptor in PC3-ARwt-Zellen darstellt.

Das folgende Beispiel illustriert die Erfindung näher, ohne sie jedoch darauf zu beschränken.

### Beispiel 1:

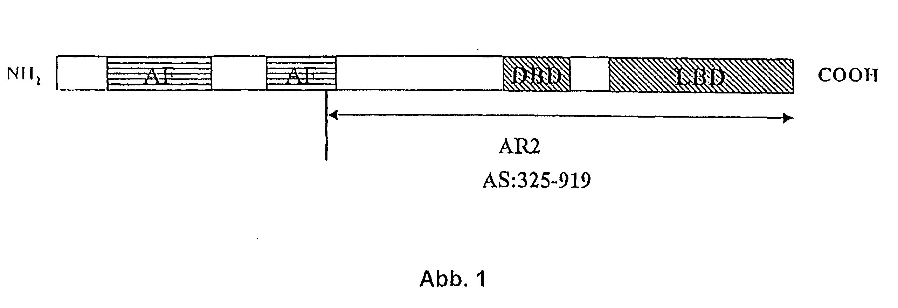

Unter Verwendung einer cDNA-Bibliothek aus fetalem Gehirn (Clontech MATCHMAKER) und eines humanen AR-Fragments, das für die Aminosäuren 325 bis 919 kodiert, als Sonde (Abbildung 1) wurde ein Screening mittels eines üblichen zwei-hybrid Hefe-Systems in Anwesenheit von Androgen 10⁻⁶M DHT durchgeführt. In Übereinstimmung mit den Anweisungen des Herstellers (Clontech) betrug die Zahl der gescreenten Klone 2x10⁷. Die Zahl der unabhängigen Klone betrug laut Angaben des Herstellers 3,5x10⁶. Davon wurden 300 positive Klone ausgewählt und mit einem β-Galaktosidase-Assay getestet, wobei 40 als lacZpositive Klone bestätigt wurden. Die Inserts dieser Klone wurden durch PCR amplifiziert. Mittels Restriktionsfragmentanalysen und Sequenzierung wurden mindestens 39 verschiedene Klone identifiziert. Einer davon war ein Klon mit einem 1110 bp umfassenden Insert (986 bp - 2096 bp), das für einen Teil des ORF (Open reading frame) von HSRNAAM kodiert (Genbank-Zugangsnummer X99209).

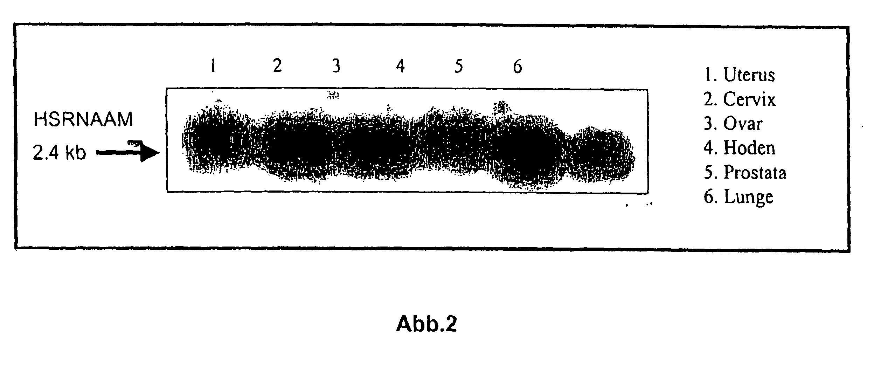

Das aus 499 bp bestehende Fragment (986 bp - 1485 bp) der HSRNAAM-cDNA Sequenz diente als Sonde für humane Blots. Die Gewebsverteilung der Arginin-Methyltransferase wurde mittels eines Northern Lights Humane multiple mRNA Blot (Life Technologies) bestimmt (Abbildung 2). Unter Verwendung der HSRNAAM Probe wurde ein Transkript entdeckt (Abbildung 2).

Abbildung 2 zeigt die Gewebsverteilung von HSRNAAM, die in an sich bekannter Weise mittels Northern-Blot-Analyse untersucht wurde. Aus verschiedenen humanen Geweben isolierte Poly-A⁺-RNA, die auf β-Aktin normalisiert wurde, wurde mit einem Formaldehyd-haltigem Agarose-Gel aufgetrennt, auf eine Nylonmembran geblottet und mit einem nomalisierten HSRNAAMcDNA-Fragment (986 - 1485 bp) hybridisiert. Ein Transkript wurde in verschiedenen humanen Geweben entdeckt (Abbildung 2).

Das mittels PCR und spezifischer Primer aus dem Hefevektor amplifizierte HSRNAAM cDNA Fragment von 986 - 1485 bp (AS 271 - 433) wurde mit den Endonukleasen *Eco*RI und *Xho*I in üblicher Weise in den Vektor pCMV-NFκB kloniert und das mittels spezifischer Primer aus humaner universal cDNA (Clontech) amplifizierte HSRNAAM cDNA Fragment von 177 bp bis 1485 bp (AS 1-433) wurde mit den Endonukleasen *Eco*RI und *Xho*I in üblicher Weise in den Vektor pCMX kloniert und mit MMTV-Luciferase in PC3-ARwt-Zellen, die den AR exprimieren, ebenfalls in üblicher Weise transfiziert.

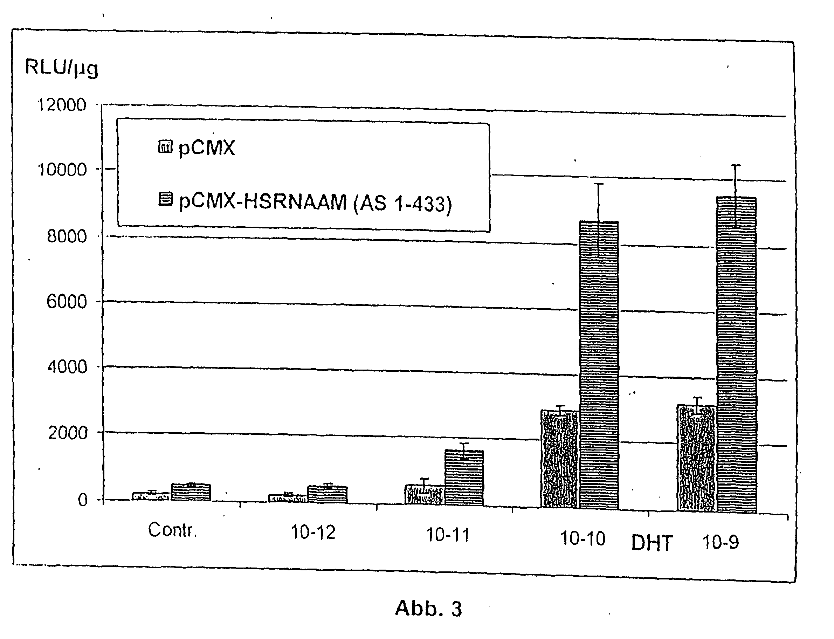

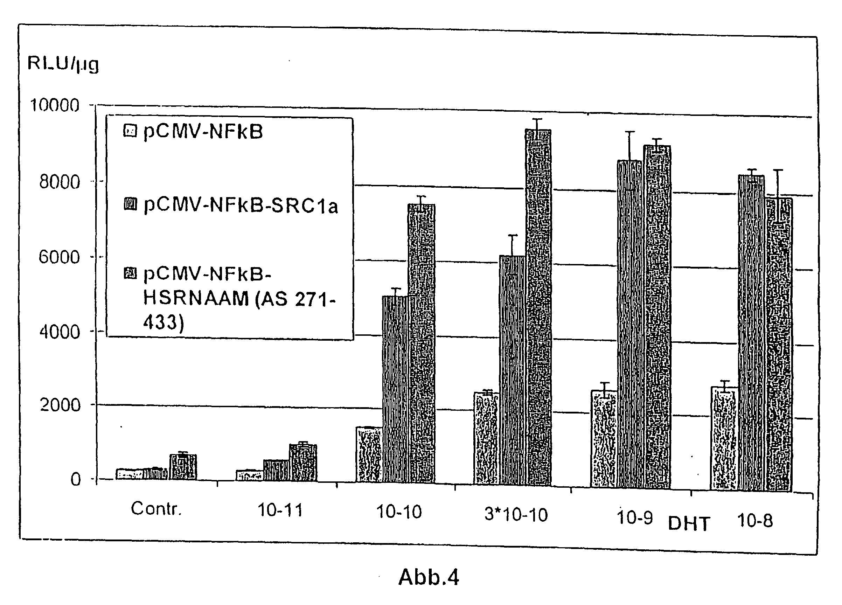

Wie Abbildung 3 entnommen werden kann, führte die transiente Transfektion von HSRNAAM-cDNA (AS 1-433) in PC3-ARwt-Zellen zu einer starken Co-Modulierung der AR-Signaltätigkeit und die Arginin-Methyltransferase wirkt somit als Co-Aktivator für den nuklearen Rezeptor. Weiterhin führte die transiente Transfektion des HSRNAAM-cDNA-Fragmentes (AS 271-433) fusioniert in frame mit der Transaktivierungsdomäne von NFκB in PC3-ARwt-Zellen zu einer starken Co-Aktivierung der AR-Signaltätigkeit (Abbildung 4), was eine Interaktion zwischen der Arginin-Methyltransferase und den nuklearen Rezeptoren oder anderen Co-Modulatoren anzeigt. Dazu wurden jeweils in eine Zellkulturschale mit Vertiefungen 2x10⁵- Zellen pro Vertiefung mit 1,0 µg MMTV-Luciferaseplasmid und 0,5 µg des Konstruktes pCMX-HSRNAAM (AS 1-433) bzw. als Negativkontrolle 0,37 µg pCMX transfiziert (Abbildung 3). In Abbildung 4 wurden 1,0 µg MMTV-Luciferaseplasmid und 0,39 µg des Konstruktes pCMV-NFκB-HSRNAAM (AS 271-433) bzw. als Negativkontrolle 0,35 µg pCMV-NFxB oder als Positivkontrolle 0,5 µg pCMV-NFkB-SRC1a transfiziert. Die transfizierten Zellen wurden 24 Stunden mit Dihydroxytestosteron (DHT) in den angegebenen Konzentrationen behandelt und nach weiteren 24 Stunden geerntet, bevor die Aktivität des Reportergens (Luziferase) gemessen wurde. Zusätzlich wurde zur Normierung die Gesamtzellproteinmenge bestimmt. Pro Transfektionsansatz und Substanzkonzentration wurden zwei Experimenten und jeweils drei Messungen durchgeführt. Die Fehlerabweichung wurde als SD angegeben. Die Aktivität ist in relativen Einheiten angegeben.

## Patentansprüche

1. Verfahren zur Testung des hormonellen Effekts, insbesondere des androgenen oder antiandrogenen Effekts, von Substanzen, bei dem
a) Zellen, die mit zwei Vektoren transfektiert sind, wobei der eine Vektor DNA, die für ein nukleäres Rezeptor-Protein oder ein Fragment davon kodiert, und der andere Vektor DNA enthält, die für den Co-Modulator HSRNAAM (Genbank X99209) oder ein Fragment davon kodiert, der Substanz ausgesetzt werden und
b) die Transkriptionsaktivität, die der nukleäre Rezeptor oder dessen Fragment in Anwesenheit von HSRNAAM oder dessen Fragment auslöst, und/oder der Einfluß der Substanz auf die Interaktion zwischen dem Rezeptor oder dessen Fragment und HSRNAAM oder dessen Fragment durch Protein-Protein-Interaktion oder Protein-Protein-DNA-Interaktion gemessen wird,
wobei unter Fragment jeweils solche Proteine verstanden werden, die eine Aminosäure oder mehrere Aminosäuren weniger als die Proteine in voller Länge und noch die funktionellen Eigenschaften eines nukleären Rezeptors oder eines Co-Modulators aufweisen.

2. Verfahren nach Anspruch 1, wobei das Fragmenten des Co-Modulators die Aminosäuren 271 bis 433 von HSRNAAM aufweist.

3. Verfahren nach einem der vorhergehenden Ansprüche, wobei der nukleäre Rezeptor ausgewählt ist unter Androgenrezeptor, Estrogenrezeptor α, Estrogenrezeptor β, Progesteronrezeptor A, Progesteronrezeptor B, Glukokortikoidrezeptor, Mineralokortikoidrezeptor, Schilddrüsenhormonrezeptor, Vitamin-D-Rezeptor, Peroxisomproliferator-aktivierter Rezeptor, Retinsäurerezeptor, Retinoid-X-Rezeptor und Orphan-Rezeptoren.

4. Verfahren nach einem der vorhergehenden Ansprüche, wobei die Zellen etablierte Zelllinien und/oder eukaryotische Zellen sind.

5. Verfahren nach Anspruch 4, wobei die eukaryotischen Zellen unter Prostatazellen, Nervenzellen, Gliazellen, Fibroblasten, Blutzellen, Osteoblasten, Osteoklasten, Hepatozyten, Epithelzellen oder Muskelzellen ausgewählt sind.

6. Verfahren nach einem der vorhergehenden Ansprüche, wobei der Vektor ein eukaryotischer Expressionsvektor ist.

7. Verfahren zur Bestimmung von Störungen im Co-Modulationsmechanismus zwischen Androgenrezeptoren und HSRNAAM (Genbank X99209), wobei die Konzentrationen von HSRNAAM oder einem Fragment davon und/oder Androgenrezeptor oder einem Fragment davon gemessen werden, und wobei unter Fragment jeweils solche Proteine verstanden werden, die eine Aminosäure oder mehrere Aminosäuren weniger als die Proteine in voller Länge und noch die funktionellen Eigenschaften eines unkleären Rezeptors oder eines Co-Modulators aufweisen.

8. Verfahren nach Anspruch 7, wobei die Konzentrationsmessung durch Radioimmunassay, ELISA-Tests, Immunfärbung, RT-PCR, Western-Blot oder Northern-Blot erfolgt.

## Claims

1. A method for testing the hormonal effect, in particular the androgenic or antiandrogenic effect, of substances, said method comprising the steps of:
a) exposing cells transfected with two vectors to the substance, wherein one of the two vectors contains a DNA, which codes for a nuclear receptor protein, or a fragment of said nuclear receptor protein, and another of the two vectors contains a DNA, which codes for an HSRNAAM co-modulator (gene bank X99209), or a fragment of said HSRNAAM co-modulator and
b) measuring transcription activity, which said nuclear receptor, or said fragment of said nuclear receptor, activates or releases in the presence of HSRNAAM or said fragment of said HSRNAAM, and/or the influence of the substance on an interaction between said receptor, or said fragment of said receptor, and HSRNAAM, or said fragment of said HSRNAAM, by protein-protein interaction or by protein-protein-DNA interaction, wherein fragment refers in each case to proteins which have one or more amino acids fewer than the full length proteins and still have the functional properties of a nuclear receptor or of a co-modulator.

2. The method as defined in claim 1, wherein said fragment of said HSRNAAM co-modulator has amino acids 271 to 433.

3. The method as defined in one of the preceding claims, wherein said nuclear receptor is selected from the group consisting of androgen receptor, oestrogen receptor α, oestrogen receptor β, progesterone receptor A, progesterone receptor B, glucocorticoid receptor, mineral corticoid receptor, thyroid gland hormone receptor, vitamin-D receptor, peroxisome proliferator-activated receptor, retinic acid receptor, retinoid X receptor and orphan receptors.

4. The method as defined in one of the preceding claims, wherein said cells are established cell lines and/or eukaryotic cells.

5. The method as defined in claim 4, wherein said eukaryotic cells are selected from the group consisting of prostate cells, nerve cells, glia cells, fibroblast cells, blood cells, osteoblast cells, osteoclast cells, hepatocytes, epithelial cells and muscle cells.

6. The method as defined in one of the preceding claims, wherein said vector is a eukaryotic expression vector.

7. A method for determining interference in a co-modulation mechanism between androgen receptors and HSRNAAM (gene bank X99209), said method comprising measuring the concentrations of HSRNAAM, or a fragment thereof, and/or androgen receptor, or a fragment thereof, wherein fragment refers in each case to proteins which have one or more amino acids fewer than the full length proteins and still have the functional properties of a nuclear receptor or of a co-modulator.

8. The method as defined in claim 7, wherein said measuring of said concentration and/or said concentrations takes place by radioimmunoassay, ELISA test, immunodyeing, RT-PCR, Western Blot or Northern Blot.

## Revendications

1. Procédé pour tester l'effet hormonal, en particulier l'effet androgène ou anti-androgène, de substances, selon lequel
a) des cellules, qui sont infectées avec deux vecteurs, dont l'un des vecteurs contient un ADN qui code pour une protéine de récepteur nucléaire ou un fragment de celle-ci, et l'autre vecteur contient un ADN, qui code pour le co-modulateur HSRNAAM (banque génétique X99209) ou un fragment de celui-ci, sont exposées à la substance et
b) l'activité de transcription, générée par le récepteur nucléaire ou par un fragment de celui-ci en présence de la HSRNAAM ou d'un fragment de celle-ci, et/ou l'influence de ladite substance sur l'interaction entre le récepteur ou un fragment de celui-ci et la HSRNAAM ou un fragment de celle-ci est/ou sont mesurée(s) par interaction protéine-protéine ou par interaction protéine-protéine-ADN, le terme fragment désignant dans tous les cas des protéines qui présentent un ou plusieurs acides aminés en moins que les protéines dans leur longueur totale tout en présentant encore les propriétés fonctionnelles d'un récepteur nucléaire ou d'un co-modulateur.

2. Procédé selon la revendication 1, **caractérisé en ce que** le fragment du co-modulateur présente les acides aminés 271 à 433 de la HSRNAAM.

3. Procédé selon l'une des revendications précédentes, **caractérisé en ce que** le récepteur nucléaire est choisi parmi le récepteur d'androgènes, le récepteur α d'oestrogènes, le récepteur β d'oestrogènes, le récepteur A de la progestérone, le récepteur B de la progestérone, le récepteur de glucocorticoïdes, le récepteur de minéralocorticoïdes, le récepteur de l'hormone thyroïdienne, le récepteur de la vitamine D, le récepteur activé du proliférateur de peroxysome, le récepteur de l'acide rétinoïque, le récepteur du rétinoïde X, ainsi que des récepteurs orphelins.

4. Procédé selon l'une des revendications précédentes, **caractérisé en ce que** les cellules sont des lignées établies de cellules et/ou des cellules eucaryotes.

5. Procédé selon la revendication 4, **caractérisé en ce que** les cellules eucaryotes sont choisies parmi les cellules prostatiques, les cellules nerveuses, les cellules gliales, les fibroblastes, les cellules sanguines, les ostéoblastes, les ostéoclastes, les hépatocytes, les cellules de l'épithélium ou les cellules des muscles.

6. Procédé selon l'une des revendications précédentes, **caractérisé en ce que** le vecteur est un vecteur eucaryote d'expression.

7. Procédé pour la détermination de perturbations dans le mécanisme de co-modulation entre des récepteurs d'androgènes et la HSRNAAM (banque génétique X99209), **caractérisé en ce que** les concentrations en HSRNAAM ou en un fragment de celle-ci et/ou en récepteur d'androgènes ou en un fragment de celui-ci sont mesurées
et **en ce que** le terme fragment désigne dans tous les cas des protéines qui présentent un ou plusieurs acides aminés en moins que les protéines dans leur longueur totale, tout en présentant encore les propriétés fonctionnelles d'un récepteur nucléaire ou d'un co-modulateur.

8. Procédé selon la revendication 7, **caractérisé en ce que** la mesure des concentrations s'effectue par méthode de radioimmunologie, par tests ELISA, par immunocoloration, par RT-PCR, par analyses Western-Blot, par Northern-Blot ou analogues.
